(19) Europäisches Patentamt
European Patent Office
Office européen des brevets

(11) **EP 4 173 559 A1**

(12) **EUROPEAN PATENT APPLICATION**

(43) Date of publication:
**03.05.2023 Bulletin 2023/18**

(21) Application number: **21205172.6**

(22) Date of filing: **28.10.2021**

(51) International Patent Classification (IPC):
**A61B 5/0507** (2021.01)     **A61B 5/00** (2006.01)

(52) Cooperative Patent Classification (CPC):
**A61B 5/0507; A61B 5/4312; A61B 5/7203**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Micrima Limited**
**Bristol BS2 0EL (GB)**

(72) Inventors:
• **CORBETT, Brandon John**
  **Bristol, BS2 0EL (GB)**
• **GIBBINS, David Rhys**
  **Bristol, BS2 0EL (GB)**
• **TSUI, Chun Sing Louis**
  **Bristol, BS2 0EL (GB)**

(74) Representative: **Haseltine Lake Kempner LLP**
**One Portwall Square**
**Portwall Lane**
**Bristol BS1 6BH (GB)**

(54) **MEDICAL EXAMINATION SYSTEM WITH SIGNAL ARTEFACT COMPENSATION**

(57) There is disclosed a method of compensating for unwanted artefacts in signal data generated by a microwave-based medical examination system which is to be used for medical imaging purposes, for example. The method decomposes a one-dimensional output signal which represents the microwave energy detected at an antenna element of the system. Individual components of the output signal are compared against predetermined reference data to determine whether they correspond to a signal artefact or a region of interest within the space. A relative intensity of subspace components that correspond to the region of interest is increased relative to an intensity of a subspace component that corresponds to the signal artefact, thereby generating a compensated one-dimensional output signal. The result is a method that can identify and remove signal artefacts produced by the system, thereby improving the quality of the acquired data and consequently the final image quality.

Figure 2

Figure 10

**Description**

**Field of the invention**

The invention relates to the field of medical examination systems, and in particular to microwave-based antenna systems for examining the internal structure of the human body. Specifically, the invention relates to a method of compensating for unwanted artefacts in signal data which is generated by such systems.

**Background**

Various medical examination systems are known for examining, e.g. imaging, the human body. Such examination techniques may be used to interrogate tissues and organs in order to identify abnormalities, such as tumours or lesions. For example, X-ray (mammography), microwave, ultrasound, MRI are all common imaging modalities.

Microwave-based examination systems typically comprise a set of one or more antenna elements which is located in operative proximity to a patient, whose anatomical tissue structure is to be imaged or otherwise investigated. A first antenna element of the set transmits microwave energy towards the patient so as to irradiate the patient, and the first antenna element or a second antenna element of the set detects the transmitted microwave energy returned back from the patient. The transmitted signal will have been scattered as a result of interactions with different anatomical regions or points within the patient, and the internal anatomy of the patient can be investigated and imaged based on an analysis of the returned signal.

A common problem with many forms of microwave-based medical examination systems is that the returned signal will include unwanted artefacts that appear as noise within the final image data and can therefore misrepresent or hide anatomical regions of interest. A common type of signal artefact is referred to as a direct coupling (DC) artefact, which is created when the microwave signal transmitted to the patient is detected by the receiving antenna directly, i.e. when the microwave signal has travelled the shortest possible range between a transmitting antenna and a receiving antenna of the system.

A standing industry practice to reduce the DC artefact is to use a method known in the art as *"coherent average subtraction"*, as described by R. C. Conceição, J. J. Mohr, and M. O'Halloran on pages 49-50 of "An Introduction to Microwave Imaging for Breast Cancer Detection", July, 2016. The system is used to acquire image data sets for the patient multiple times from different viewpoints, while the distance separating the transmit and receive antenna elements is kept constant. As the positional relationship between antenna elements remains the same for each data set, the direct coupling artefacts will appear as coherent signals across the data sets. In contrast, the signals arising from interaction with the anatomy of the patient will be incoherent across the data sets. Accordingly, any averaging of the data sets will yield the coherent signal arising from the DC artefact, and this is then subtracted out of the individual data sets that are to be used to generate the image.

Coherent average subtraction can be useful to reduce unwanted artefacts. However, its usefulness is limited to removing artefacts that appear coherent from different acquisition viewpoints. Further, in breast cancer imaging applications, the medical examination system will typically comprise a rotatable set of antenna elements which surround a breast to be investigated. In those arrangements, the signals returned from the centre of the breast, i.e. close to the axis of rotation of the set of antenna elements, will be coherent because they will appear at the same range (distance) from the antenna element(s) in each dataset. Therefore, coherent average subtraction of that data will cause this central area to be removed from the final image data despite the fact that it corresponds to an anatomical region of interest.

Accordingly, it is desirable to provide an improved method of compensating for unwanted artefacts in signal data generated by a microwave-based medical examination system.

**Summary**

According to an aspect, there is provided a method, in a microwave-based medical examination system comprising a set of one or more antenna elements in operative proximity to a space which includes an object to be investigated, wherein the method comprises:

energising a first antenna element of the set to transmit microwave energy towards the space so as to irradiate the object;
detecting, at the first antenna element or a second antenna element of the set, the transmitted microwave energy returned back from the space including the object;
generating a one-dimensional output signal representing the detected microwave energy;
separating the one-dimensional output signal into a plurality of subspace components;
determining, for each one of the plurality of subspace components, whether the subspace component corresponds to a signal artefact or a region of interest within the space, based on a comparison of the subspace component to predetermined reference data; and

processing the one-dimensional output signal to increase a relative intensity of one or more subspace components that have been determined to correspond to the region of interest, wherein the relative intensity is with respect to an intensity of a subspace component that has been determined to correspond to the signal artefact, thereby generating a compensated one-dimensional output signal.

The one-dimensional output signal may represent the scattering parameters of the detected microwave energy.

The plurality of subspace components, which are compared to the reference data for the determination step, may be all of the subspace components into which the one-dimensional (1D) output signal has been divided. This, however, is not required. The step of determining whether the subspace component corresponds to a signal artefact or a region of interest may be performed with respect to a subset of the total number of subspace components into which the 1D output signal is divided. In those arrangements, the plurality of subspace components may be a subset of the total number of subspace components of the 1D output signal.

The region of interest may be any region or point within the space that is to receive the object. The region of interest may be on the surface of, or may be within, the object, or both. In optional embodiments, the object is an anatomical object and the region of interest is a region of the object at which a tumour or other pathology is expected.

Separating the one-dimensional output signal into a plurality of subspace components may comprise: generating a matrix representation of the one-dimensional output signal; performing a singular value decomposition to factorise the matrix in terms of its singular vectors and a group of singular values; generating, for each singular value of the group, a component matrix by reversing the singular value decomposition with respect to the singular value, wherein each component matrix represents a respective subspace component of the one-dimensional output signal. Optionally, the method further comprises concatenating the matrix representation.

The matrix representation may be a Hankel matrix representation.

Determining whether the subspace component corresponds to a signal artefact or a region of interest may comprise: generating a range profile of the subspace component; comparing the range profile to the reference data; and concluding that the subspace component corresponds to the signal artefact or the region of interest based on whether or not the range profile satisfies a match criterion indicating that it is sufficiently similar to the reference data.

The reference data may be in the form of a reference range profile for the signal artefact or the region of interest. The reference range profile may be generated based on a model of the signal response arising from the signal artefact. Comparing the range profile to the reference data may comprise performing a cross-correlation of the range profile and the reference range profile, to generate a cross-correlated profile. The range profile may satisfy the match criterion if the cross-correlated profile has a peak location within a predefined threshold displacement range from an origin of the cross-correlated domain.

The reference data may be in the form of a reference range value for the signal artefact or the region of interest. The range profile may satisfy the match criterion if the range profile has a peak location within a predefined threshold displacement range from the reference range value.

The predefined threshold displacement range may be calculated as: $\dfrac{v}{B}$, where B is the bandwidth of the transmitted microwave energy and v is its speed of propagation.

Processing the one-dimensional output signal to increase a relative intensity of one or more subspace components that have been determined to correspond to the region of interest may comprise: associating the plurality of subspace components with respective weighting factors, wherein the weighting factor for a subspace component that has been determined to correspond to the signal artefact is less than the weighting factor for a subspace component that has been determined to correspond to the region of interest; applying the weighting factors to the plurality of subspace components to generate a plurality of weighted subspace components; and combining the plurality of weighted subspace components to generate the compensated one-dimensional output signal.

The weighting factors may be applied to the subspace components by multiplying each subspace component (i.e. the $\Phi_l$ values) by its associated weighting factor. The weighted subspace components may then be summed according to equation 8 (where $\Phi_l$ values of equation 8 are replaced by weighted values of $\Phi_l$).

Where the 1D output signal is separated by performing a singular value decomposition to factorise a matrix representation of the 1D output signal, the step of processing the one-dimensional output signal to increase a relative intensity of one or more subspace components that have been determined to correspond to the region of interest may comprise: associating the plurality of subspace components with respective weighting factors, wherein the weighting factor for a subspace component that has been determined to correspond to the signal artefact is less than the weighting factor for a subspace component that has been determined to correspond to the region of interest; applying the weighting factors to the group of singular values to generate a weighted group of singular values; and reversing the singular value decomposition by combining the singular vectors and the weighted group of singular values. This may be done according to equations, 11, 12 and/or 13.

The weighting factor for the subspace component may be calculated based on an extent of similarity between the subspace component and the reference range profile. Where the reference data is in the form of a reference range profile for the signal artefact, the weighting factor for the subspace component that has been determined to correspond to the signal artefact may be inversely related to an extent of similarity between the subspace component and the reference range profile for the signal artefact.

The range profile and the reference range profile may be normalised prior to the cross-correlation. The weighting factor for the subspace component that has been determined to correspond to the signal artefact may be calculated as: $1 - I_l$, wherein $I_l$ is an intensity value of a peak in the cross-correlated profile.

The transmitted microwave energy may be detected at the second antenna element. The predetermined reference data may represent an aspect of the microwave energy that is expected to be detected at the second antenna element as a result of direct coupling between the first antenna element and the second antenna element.

The reference data may comprise a reference range profile in the form of a point spread function centred at a predetermined range at which the signal artefact or the region of interest is expected. The one-dimensional output signal may represent the scattering parameters of the detected microwave energy.

The one-dimensional output signal may represent the microwave energy detected by the first antenna element or the second antenna element only, during an acquisition period in which only the first antenna element transmits the microwave energy.

According to another aspect, there is provided a microwave-based medical examination system comprising:

> a set of one or more antenna elements in operative proximity to a space for receiving an object to be imaged; and
> processing circuitry configured to perform the method of any preceding statement.

The medical examination system may have any one or more or all of the features described above.

The Applicant has recognised that the steps of separating a one-dimensional output signal into a plurality of subspace components and adjusting the intensities of one or more of the subspace component (thereby generating a compensated one-dimensional output signal) may be novel and inventive in its own right. That is, the method described above need not be limited to applications in which the components are modified to compensate for signal artefacts, specifically; the method may instead be used to perform any component level adjustment as may be required or desirable for the specific application for which the method is to be used.

Thus, according to another aspect of the invention, there is provided a method, in a microwave-based antenna system comprising a set of one or more antenna elements in operative proximity to a space which includes an object to be investigated, wherein the method comprises: energising a first antenna element of the set to transmit microwave energy towards the space so as to irradiate the object; detecting, at the first antenna element or a second antenna element of the set, the transmitted microwave energy returned back from the space; generating a one-dimensional output signal representing the detected microwave energy; separating the one-dimensional output signal into a plurality of subspace components; and processing the one-dimensional output signal to adjust an intensity of one or more subspace components, thereby generating a compensated one-dimensional output signal. According to this aspect, the present invention provides a versatile and sophisticated method of accessing and adjusting signal components.

The one-dimensional output signal may be separated into a plurality of subspace components according to any one of the methods described herein. For example, a singular value decomposition may be performed on a (e.g. Hankel) matrix representation of the one-dimensional output signal. The intensities of the components may be adjusted in any suitable manner, e.g. by applying weighting factors to the subspace components or their associated singular values. Further, the plurality of (e.g. adjusted or weighted) components may be recombined to form a compensated one-dimensional output signal using any one of the methods described herein, such as by using equation 8 or equation 12 and optionally equation 13.

It will be appreciated that all of the above aspects of the present invention extend to radiofrequency antenna system generally. For example, the system need not operate in the microwave range of the electromagnetic spectrum. Further, it is not essential for the system to be limited to medical examination applications.

## Brief description of the drawings

For a better understanding of the invention, and to show more clearly how it may be carried into effect, reference will now be made, by way of example, to the accompanying drawings, in which:-

> Figure 1 is a system diagram of a microwave-based medical examination system according to an embodiment of the invention;

Figure 2 is a schematic view of the medical examination system;

Figure 3 is a flowchart depicting a sampling method;

Figure 4 is a flowchart illustrating a method of compensating for unwanted direct coupling artefacts, in accordance with an embodiment of the present invention;

Figure 5 is a graph which schematically illustrates a one-dimensional output signal generated by the method of Figure 3, wherein the one-dimensional output signal is represented in the frequency domain;

Figure 6 is a graph which schematically illustrates the one-dimensional output signal in the time domain;

Figure 7 is a flow diagram schematically illustrating a method for separating the one-dimensional output signal into its subspace components, in accordance with an embodiment of the present invention;

Figure 8 is a graph which schematically illustrates the range profile of two components identified by applying the method of Figure 7 to simulated data;

Figure 9 is a plot schematically illustrating a reference range profile for a direct coupling artefact model;

Figure 10 is a graph schematically illustrating the result of a cross-correlation between the two components identified above with respect to Figure 8 and the range profile for the direct coupling artefact model; and

Figure 11 is a plot showing the range profile of a compensated one-dimensional output signal generated by applying the method of the present invention to the simulated data.

## Detailed Description

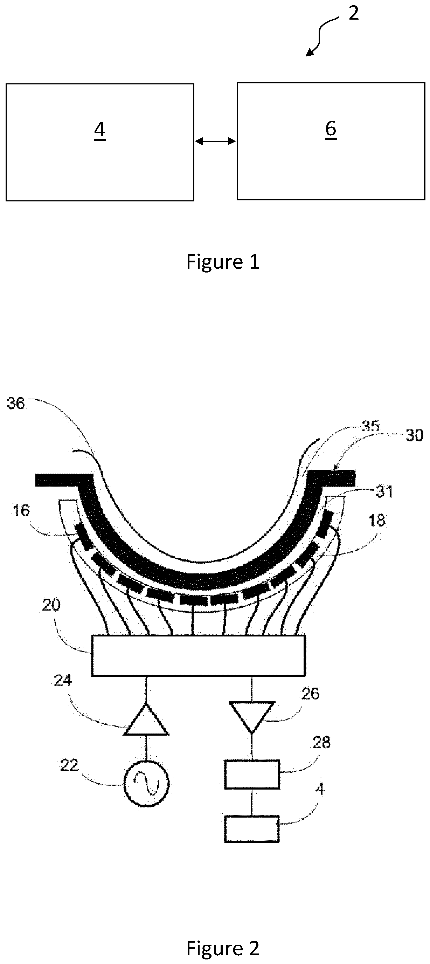

**Figure 1** shows a microwave-based medical examination system 2 according to an embodiment of the invention. The medical examination system generally comprises a processor 4 and an antenna array 6 in communication with the processor 4.

As shown in **Figure 2**, the antenna array 6 comprises a set of plural N (e.g. 60) antenna elements (also referred to herein simply as "antennas") 16 which are statically positioned over the surface of, or within, a substrate 18. In this example embodiment, the medical examination system 2 is to be used as an imaging modality which generates image data representing the internal structure of a female breast, e.g. to detect suspicious lesions that may be indicative of breast cancer. For that reason, the shell 18 has a curved profile as shown. In particular, the shell 18 is part or hemi-spherical and is configured to approximate the shape of a breast. The antennas 16 are arranged over the shell 18 such that they all point to a common focal point in a space which receives the breast 36. The shell 18 and thus the antenna array 6 may have a different profile, as may be appropriate for the specific application of the medical examination system.

The antennas 16 are each electrically connected to a switching matrix 20. The switching matrix 20 is in turn connected to both a transmit path and a receive path. The transmit path comprises a signal generator 22 coupled to an amplifier 24. The receive path comprises an amplifier 26 coupled to a detector 28 and the processor 4. The switching matrix 20 selectively couples each of the antennas 16 to either the transmit path or the receive path. In other embodiments, however, the switching matrix may be omitted, and the antennas may instead be connected to respective ports of the transmit path and the receive path.

In the present embodiment, the antenna array 6 is operated in a multi-static fashion. Specifically, the switching matrix 20 is controlled so as to connect one of the antennas 16 to the transmit path and the remaining antennas 16 to the receive path. The signal generator 22 generates a stepped frequency continuous wave (CW) signal which is amplified by the amplifier 24 and then transmitted by the antenna 16 connected to the transmit path. The stepped frequency continuous wave signal is a sequential series of pulses of continuous radiofrequency wave energy, where each pulse has its frequency stepped up across a range of frequencies. The range of frequencies is within the microwave range of the electromagnetic spectrum, typically 3-8 GHz. The other antennas 16 receive and detect the transmitted radiofrequency wave energy and the received signal is recognised and then recorded by the processor 4.

As shown in Figure 2, the shell 18 receives a cup 30. The cup 30 has a complementary shape to the shell 18 such that if fits snugly within the shell 18. A layer of coupling fluid (dielectric constant controlled fluid) may be inserted in the gap 31 between the shell 18 and the cup 30 so as to improve the coupling between the antennas 16 and the cup 30 in order to minimise signal loss and thus improve transmission of the microwave signal.

An actuator (not shown), such as a motor, may be connected to the microwave antenna array 6. The actuator is configured to move the microwave antenna array 6 relative to the cup 30 which remains stationary against the breast 36. Specifically, the actuator causes the microwave antenna array 6 to rotate relative to the cup 30 about the breast 36, thereby allowing for multiple sampling positions. The microwave antenna array 6 rotates about the centre of the shell 18 (i.e. its axis of rotation). The array is received in a housing (not shown), within which the array is rotatable. A top surface of the housing has pins for engaging corresponding holes in a flanged portion of the cup, such that the housing and the cup are fixed relative to one another during operation of the array. The cup 30 may be removed so as to enable the coupling fluid to be replaced. Other known mechanisms for rotating the antenna array 6 could be used instead.

The antennas 16 may be as described in WO 2009/060181. In particular, the antennas 16 may comprise a slot 16 formed in a conductive element, the slot having a rectangular external boundary defined by a substantially closed internal edge of the conductive element. A microstrip feed line may be spaced from the conductive element by a dielectric substrate with the distal end of the line positioned at the geometric centre of the slot.

In use, a patient lies motionless in a prone position such that their breast 36 sits in the cup 30 and remains at a fixed position relative to the antenna array during the acquisition process. A layer of coupling fluid may also be provided in the gap 35 between the cup 30 and the breast 36 so as to improve coupling between the antennas 16 and the breast 36 in order to minimise signal loss and thus improve transmission of the microwave signal.

Although not shown, one or more inserts may be placed inside the cup 30 so as to enable a better fit between the internal surface of the cup 30 and the breast 36. For example, a plurality of such inserts may be provided, each having different shapes and sizes, to enable the system to be better adapted to breasts of different shapes and sizes. The inserts may be made from the same material as the cup (e.g. ceramic).

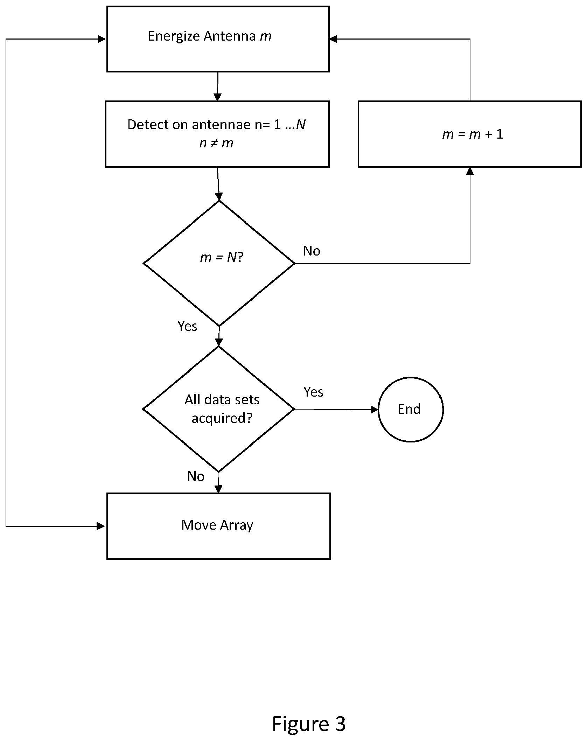

**Figure 3** shows a flowchart of a data acquisition method. The data acquisition method is divided into a plurality of acquisition periods. During each acquisition period, the antenna array 6 is at a fixed position with respect to the breast 36 under investigation and the switching matrix 20 connects a single antenna m of the N antennas to the transmit path and all other antennas 16 (n = 1...N, n≠m) are connected to the receive path. Further, during each acquisition period, the antenna 16 connected to the transmit path is energized to transmit the stepped frequency CW microwave signal towards the space which includes the breast, so as to irradiate and illuminate the breast 36 with the microwave signal. The breast being imaged may be considered as a network of tissue regions or points having variable material (e.g. dielectric) properties, and the transmitted signal interacts with and is scattered by those regions/points before the scattered signal is received at each of the non-transmitting antennas 16 where it is detected (possibly in a time-sharing arrangement) and recorded during the acquisition period.

If m≠N, the switching matrix 20 steps to the next antenna 16 (m = m + 1), and the data acquisition method begins the next acquisition period in which the next antenna 16 is connected to the transmit path and all other antennas 16 are connected to the receive path. This is repeated until all antennas 16 have been connected to the transmit path. Accordingly, the data acquisition method comprises N acquisition periods for the current position of the antenna array 6.

If additional data sets are required, then the actuator is used to rotate the array 6 relative to the breast 36 while retaining the breast in position. The data acquisition method is then repeated with the antenna array 6 in the new position.

During an acquisition period, the antenna array may be regarded as a plurality of transmit-receive antenna pairs, where each pair comprises only two antenna elements: the antenna 16 connected to the transmit path and a respective one (i.e. only one) of the antennas 16 connected to the receive path. The processor 4 generates and records a one-dimensional (1D) output signal which represents the measured (detected) microwave energy for a single transmit-receive antenna pair. A 1D output signal is generated for each pair of transmit-receive antenna elements. In the present embodiment, the 1D output signal represents scattering parameters between a single transmit-receive antenna pair. The scattering parameters have the form of a magnitude and phase change between the transmitted and received wave form, for each frequency of the stepped frequency CW signal, which is recorded as a complex number (having real and imaginary parts).

The 1D output signal may be thought of as a summation of plural signal subspace components, where each subspace component is a signal response from a respective point within the space being imaged. That is, each component corresponds to the microwave energy that has travelled along a channel from the transmitting antenna element to a point within the space and from the point to the receiving antenna element of the transmit-receive antenna pair. The 1D output signal generated for each transmit-receive antenna pair will generally comprise at least three types of signal components: a component arising from direct coupling between the transmitting and receiving antennas 16 (in which case the point is along a direct-line of sight between the antenna elements of the transmit-receive antenna pair); a component arising from radiation which reflects off a point on the skin of the breast 36 (an external tissue region); and a component arising from radiation which reflects off (i.e. is scattered by) a point corresponding to an internal tissue region within the breast. It will be appreciated that this list is not an exhaustive one and that the 1D output signal may have less than or more than the three types of signal components described above.

The 1D output signals may be used by the processor 4 to construct image data of the internal structure of the breast 36. Data reconstruction may be performed using Phased Array (frequency domain), Delay and Sum (DAS - time domain) techniques or any other suitable technique. From this, the processor 4 is able to identify (possibly, with additional user input or confirmation) a target region in which a possible tumour or other pathology.

The region of points within the breast (and/or in some cases, on the skin itself) will be the region of interest in the final image data. However, as stated above, the direct coupling component of each 1D output signal appears as an unwanted artefact in the data and can contribute significantly to degrading the quality of the final image data. The present invention therefore processes at least one (and in embodiments all) of the detected 1D output signals to dampen (i.e. reduce) the strength or intensity of (the detected microwave energy or scattering parameters that corresponds to) the direct coupling component(s) relative to that of components which correspond to the region of interest.

The present invention generates a compensated 1D output signal, wherein the intensities of components which correspond to the region of interest relative to the intensity of an artefact component, i.e. the intensities of components of interest in relation to (e.g. as a scaling factor of) the intensity of an artefact component, is larger in the compensated 1D output signal than it is in the original 1D output signal. This is advantageous in that it may improve the accuracy of the final image data and the detectability of reflections resulting from regions of interest within the breast.

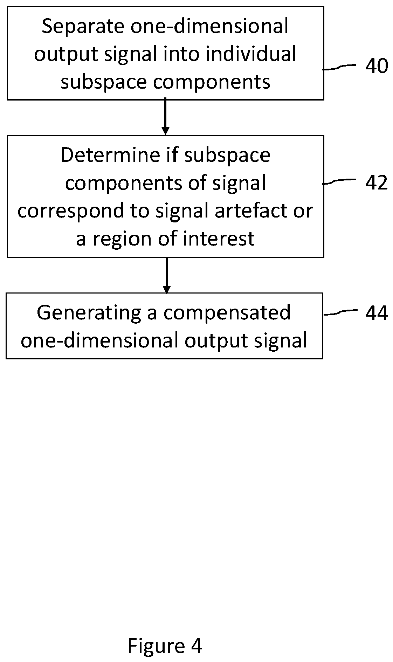

**Figure 4** is a flowchart illustrating a method of compensating for an unwanted direct coupling artefact in a 1D output signal which has been generated for a given transmit-receive antenna pair during an acquisition period, in accordance with an embodiment of the present invention.

The method begins at block 40, at which the 1D output signal is separated into its individual subspace components. As stated above, the 1D output signal generated by the medical examination system described above with respect to Figure 3 can be considered as a summation of individual complex signal components. At block 40, therefore, the 1D output signal is separated into those subspace components for further investigation.

At block 42, the method determines whether the subspace components correspond to a direct coupling signal artefact or a region of interest, such as the region within the breast. This is done based on a comparison of the subspace component to predetermined reference data.

As is described in further detail below, in preferred embodiments the reference data is predetermined for the signal artefact, and represents an aspect of the signal response (the measured microwave energy or scattering parameters) that is predicted to arise from direct coupling between the antenna pair. Each component of the 1D output signal is then compared, at block 42, to the predetermined reference data for the signal artefact. If the component satisfies a match criterion indicating sufficient similarity to the reference data, the component is determined to correspond to the direct coupling signal artefact and may be flagged as such. Otherwise, if the subspace component does not satisfy the match criterion, the component is determined to correspond to signals returned from a region of interest.

Having identified components of the 1D output signal that correspond to the direct coupling artefact, and components that correspond to a region of interest, the method proceeds to block 44, at which a compensated 1D output signal is generated. In effect, the present invention modifies the original 1D output signal by increasing a relative intensity of the subspace components that have been determined to correspond to the region of interest, wherein the relative intensity is with respect to an intensity of a subspace component(s) that has been determined to correspond to the signal artefact.

The compensated 1D output signal can then be passed to a subsequent processing stage for image processing.

By separating the original 1D output signal into its individual subspace components, comparing those components to predetermined reference data, and increasing the relative intensities of components which have been determined to correspond to the region of interest, the present invention provides a useful alternative to the conventional method of coherent average subtraction. For example, the present invention allows signal artefacts to be dampened (thereby reducing their intensities as compared to the intensities of signals arising from the region of interest) but without suffering from the above-described disadvantages of the coherent average subtraction method, such as the need to acquire data from different viewpoints, or the loss of data at the centre of an antenna array due to the effects of rotational processing of data sets.

Further, by analysing the components of an individual 1D output signal generated for a single transmit-receive antenna pair during a single acquisition period, the present invention may provide a versatile and sophisticated method that can compensate for more complex signal artefacts in the acquired data. This is especially the case when compared to hypothetical arrangements in which plural 1D output signals for respective antenna pairs (or for different acquisition periods) are combined or averaged before component analysis. For example, by processing and analysing an individual 1D output signal, the present invention is able to compensate for signal artefacts that are unique to that antenna pair, and/or compensate for signal artefacts from a point that appears at a different range from one transmit-receive antenna pair to another.

The processing steps at each block of Figure 4 will now be described in detail for each block in turn.

Separating signal into subspace components

For ease of understanding, the 1D output signal (scattering parameters) can be generalised to comprise of P signal responses from unique points within the irradiated space at a range $R_p$ between the transmit and receive antennas of a single transmit-receive antenna pair, as follows:

$$\Phi(n) = \sum_{p=1}^{P} \frac{A_p}{R_p^2} e^{-i\frac{2\pi f(n)}{v} R_p} \qquad (1)$$

where, Ap is the amplitude/intensity of the signal response from the $p_{th}$ point, $f(n)$ is the nth transmitted frequency of the stepped frequency CW range described above with respect to Figure 3, and *v is* the wave velocity of the transmitted microwave signal.

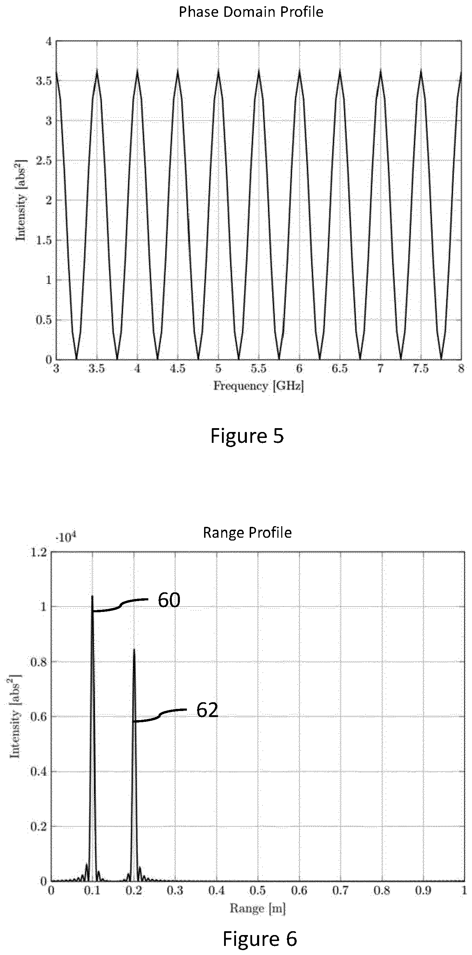

**Figure 5** is a graph which schematically illustrates the 1D output signal, $\Phi$, generated based on data for a simulated measurement of the signal response from two isotropic point scatterers at a range $R_p$ of 0.1m and 0.2m, respectively. The output signal comprises plural intensity values for respective transmitted frequencies across a bandwidth range of 3-8 GHz. In the present embodiment, the 1D output signal is generated (and shown in Figure 5) in the frequency domain, although the associated time domain data may be generated instead. In either case, the 1D output signal may be transformed between the time domain and the frequency domain by applying a Fourier transform to the data as appropriate. The time domain data, which is referred to herein as a "range profile", is plotted in **Figure 6,** which shows two clear peaks 60, 62 corresponding to the isotropic point scatterers at the ranges 0.1m and 0.2m.

It will be appreciated that upon a true measured data set, the 1D output signal will comprise the signal responses from many individual points within the space including the breast being imaged, such that the individual components of the 1D output signal are not readily identifiable in the 1D output signal, e.g. by analysing its range profile. The method of the present invention therefore processes the 1D output signal to separate it into its individual subspace components, which are then analysed separately.

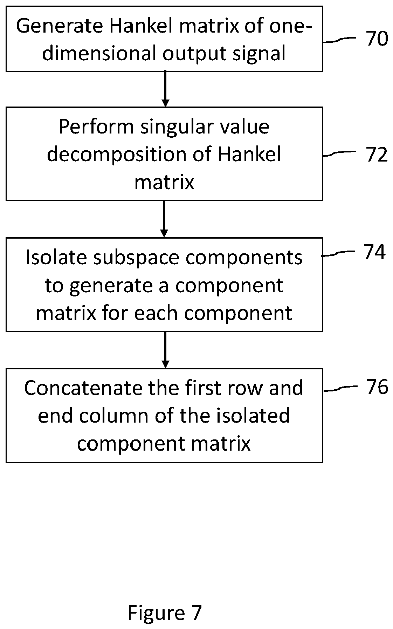

**Figure 7** is a flow diagram schematically illustrating the method steps for separating the 1D output signal into its subspace components, in accordance with an embodiment of the present invention.

The method begins at block 70, at which a matrix representation of the 1D output signal is generated. A sampling window is slid over the 1D output signal and each windowed section of data is stacked into a row of a data matrix. In this embodiment, the 1D output signal is expanded into a Hankel matrix containing multiple sub bands of data from across the entire signal bandwidth, N. The Hankel matrix, X, is represented as follows:

$$\mathbf{X} = \begin{bmatrix} \Phi(0) & \Phi(1) & \dots & \Phi(L-1) \\ \Phi(1) & \Phi(2) & \dots & \Phi(L) \\ \vdots & \vdots & \vdots & \vdots \\ \Phi(j) & \Phi(j+1) & \cdots & \Phi(j+L-1) \\ \vdots & \vdots & \vdots & \vdots \\ \Phi(J-2) & \Phi(J-1) & \cdots & \Phi(J+L-3) \\ \Phi(J-1) & \Phi(J) & \cdots & \Phi(J+L-2) \end{bmatrix} \qquad (2)$$

where the sampling window comprises a sub-bandwidth of L frequency samples of $\Phi$, *j* is the starting frequency sample of a sub-bandwidth, and J is the number of windowed sections. In the present invention, L = N/2 and *J = N-L*+1. However, these variables have the potential to be tuned for specific applications, for example L and J could both be reduced if one were interested in investigating the lower frequencies of the provided bandwidth for example.

An advantageous feature of the Hankel matrix represented by **X** is that the original 1D output signal, $\Phi$, can be fully recovered through the concatenation of the first row and end column of the matrix:

$$\Phi = ([\mathbf{X}_{1,L}, \mathbf{X}_{1,L-1}, \dots, \mathbf{X}_{1,1}], [\mathbf{X}_{2,1}, \mathbf{X}_{3,1}, \dots, \mathbf{X}_{J,1}]) \qquad (3)$$

At block 72, the Hankel matrix, X, is decomposed by applying a singular value decomposition (SVD), which factorises the matrix representation of the 1D output signal into three matrices U, S, V:

$$\text{SVD}(\mathbf{X}) = [\mathbf{U}, \mathbf{S}, \mathbf{V}] \qquad (4)$$

where **U** is an mxm unitary matrix which represents the left-hand singular vectors (of size $J \times J$), **S** is a rectangular diagonal matrix of singular values (of size $J \times L$), and V is an n×n unitary matrix that represents the right-hand singular vectors (of size $L \times L$). The decomposition may be written in summation form as follows:

$$X = \sum_{l=1}^{L} \sigma_l u_l v_l^{H} \qquad (5)$$

where $u_l$ and $v_l$ are the lth column vectors from matrices **U** and **V**, respectively, and $\sigma_l$ is the lth singular value at the $l \times l$ diagonal position of matrix **S.**

The singular values of the **S** matrix, i.e. $\sigma_l$ in equation 5, can be interpreted as corresponding to the principal components of the 1D output signal. In total there are L signal components identified from this decomposition.

At block 74, an individual subspace component is isolated and calculated in matrix form by reversing the decomposition for a respective singular value of the group of singular values in the matrix, **S,** output from the decomposition. Reversing the decomposition comprises the step of calculating the product of the **U** matrix, the complex conjugate transpose of the **V** matrix, and a single diagonal entry (singular value) in the S matrix. This process generates a unique component Hankel matrix, $X_l$, as follows:

$$X_l = \sigma_l u_l v_l^{H} \qquad (6)$$

where the unique component matrix corresponds to a respective subspace component of the one-dimensional output signal.

At block 76, the associated signal response $\Phi$ of the signal component is then extracted through a concatenation of the component matrix, as follows:

$$\Phi_l = ([X_{l1,L}, X_{l1,L-1}, ..., X_{l1,1}], [X_{l2,1}, X_{l3,1}, ..., X_{lJ,1}]) \qquad (7)$$

The first component, $\Phi_1$, is associated with the largest singular value output from the decomposition, and thus represents the feature responsible for the greatest variance within the data matrix **X** and therefore can be interpreted as carrying the greatest information/highest importance within the given data. The second component, $\Phi_2$, is associated with the second largest singular value output from the decomposition, and thus represents the feature responsible for the second greatest variance within the data matrix **X.** Accordingly, the signal components, $\Phi_l$, may be ranked by their importance within the given data in terms of their associated singular values.

As will be described in further detail below, an advantageous property of each subspace component $\Phi_l$ produced from the above-described singular value decomposition of a Hankel matrix is that the original 1D output signal can be reconstructed from a superposition of the subspace components, as follows:

$$\Phi = \sum_{l=1}^{L} \Phi_l \qquad (8)$$

This is unique to the decomposition of the Hankel matrix, and would not be achievable by hypothetical spectral estimation techniques that decompose an autocorrelation matrix of the output signal (and not a matrix of data samples directly) making data recovery not possible.

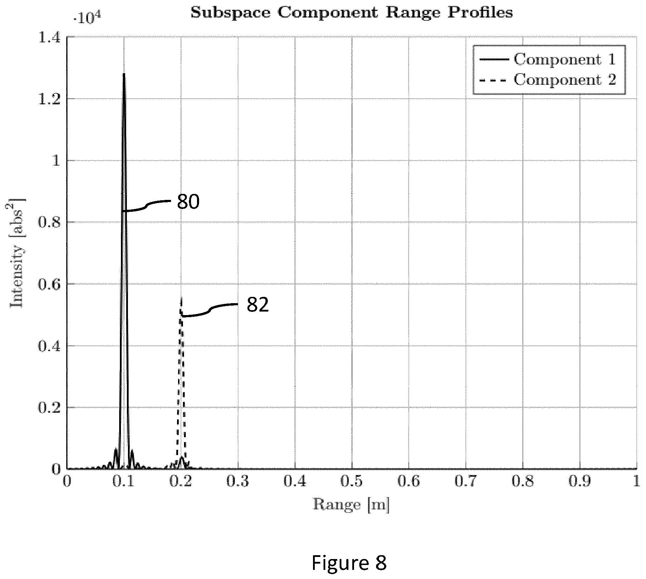

**Figure 8** is a graph which schematically shows the range profiles of components identified by applying the above-described decomposition method to the simulated response data for two isotropic point scatterers at a range $R_p$ of 0.1m and 0.2m, respectively. As can be seen in Figure 8, the decomposition method described above was able to identify the two subspace components, $\Phi_1$ and $\Phi_2$, which form two intensity peaks 80, 82 at the correct ranges of 0.1m and 0.2m, respectively.

Identifying signal artefacts

Having separated the 1D output signal into its individual subspace components, $\Phi_l$, each component is analysed (at bock 42 of Figure 4) to determine whether it corresponds to a signal artefact, or a region of interest within the space being irradiated.

As stated above, each component $\Phi_l$ is compared to a (i.e. the same) predetermined reference data for the DC signal artefact. In a first embodiment, the predetermined reference data includes a model of the signal response, $\Phi_{dc}$, arising from direct coupling between the transmit and receive antennas of the transmit-receive antenna pair, as follows:

$$\Phi_{dc}(n) = \frac{A_{dc}}{R_{dc}^2} e^{-i\frac{2\pi f(n)}{v} R_{dc}} \qquad (9)$$

where $A_{dc}$ is the amplitude (intensity) of the DC signal response, $R_{dc}$ is the two-way range between the transmit and receive antennas in question, $f(n)$ is the frequency transmitted for the nth frequency sample, and $v$ is the wave velocity of the transmitted microwave signal. The signal response corresponding to the direct coupling artefact will occur at a known line-of-sight range $R_{dc}$ for each antenna pair, i.e. the known distance along the shortest possible path between a transmitting antenna and a receiving antenna.

The comparison may be performed in any suitable way that determines an extent of similarity between the two. The comparison may be with respect to the phase domain data. In the present embodiment, however, a range profile of each subspace component is compared to a reference range profile defined by the direct coupling model. To facilitate this, the phase domain data for each detected subspace component, $\Phi_j$, and the predetermined model of the DC artefact, $\Phi_{dc}$, may be Fourier transformed into the time domain. However, if the original 1D output signal is detected and recorded in the time domain, then this Fourier transform step can be omitted for at least the subspace components.

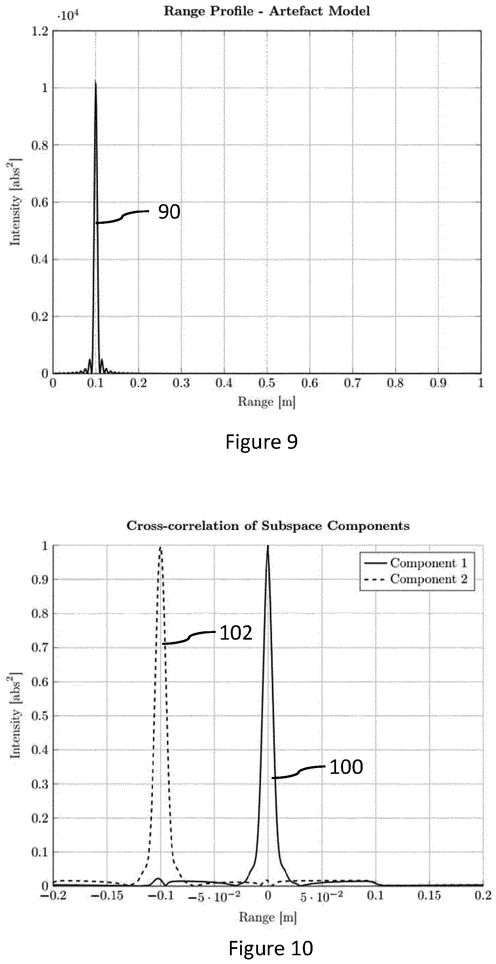

**Figure 9** is a plot showing the reference range profile forming the reference data for the DC artefact model of equation 9, where the DC signal response is assumed to be at a range $R_{dc}$ of 0.09m between transmit and receive antennas, although in practice the DC range may vary depending on the antenna array in question. As shown, the reference range profile 90 for the DC response takes the form of a point spread (sinc) function centred at the range $R_{dc}$, in this case 0.09m.

The component range profiles may be compared to the reference range profile in any suitable way that provides an indication of the extent of similarity between the component and the model, and thus whether the component satisfies a match criterion for the DC artefact. In this embodiment, each component range profile is cross-correlated against the reference range profile. To facilitate this, the intensity data of the range profiles may be normalised prior to cross-correlation and the cross-correlation may be with respect to the normalised range profiles.

The cross-correlation generates a cross-correlated profile which represents the displacement of a peak in the component range profile relative to the peak of the reference range profile, which is located at the origin of the cross-correlated domain. A component is determined to satisfy the match criterion, and thus corresponds to the DC artefact, if a peak of the cross-correlated profile is located within a predefined threshold displacement range from the origin of the cross-correlated domain. The predefined threshold displacement range, in this embodiment, is calculated as twice the resolution size of the signal, $2\frac{v}{2B} = \frac{v}{B}$, where B is the signal bandwidth and v is the speed of propagation. With respect to the simulated example signal having a 5GHz bandwidth, the predefined threshold displacement range is 0.02m.

**Figure 10** is a graph showing the cross-correlated profiles of the two components (components 1 and 2) identified above with respect to Figure 8 when overlaid on the same graph.

As can be seen, the cross-correlated peak 100 for component 1, $\Phi_1$, and the cross-correlated peak 102 from component 2, $\Phi_2$, are located at different positions from the origin (0, 0) of the cross-correlated domain. Peak 100 (component 1) falls within the predefined threshold displacement range (at 18.97mm from the origin) and so the method will identify it as a subspace component responsible for DC. The component (component 1) corresponding to peak 100 will therefore be determined to belong to the DC signal artefact. Peak 102, however, has a displacement value of-0.12 from the origin point and therefore falls outside the predefined threshold displacement range. Component 2 will therefore be determined to belong to a tissue region and be identified as a component corresponding to a region of interest.

In an alternative embodiment to that described above, the predetermined reference data does not include a model of the signal response, $\Phi_{dc}$, arising from direct coupling between the transmit and receive antennas of the transmit-receive antenna pair. Accordingly, the determination performed at block 42 of Figure 4 does not rely on a predetermined model of the direct path signal. Instead, the reference data may comprise a single reference range value at which the signal artefact is estimated to, or has otherwise been predetermined to, occur. The reference range value may indicate the position within a range profile at which the signal artefact is estimated to be. In such cases, the determination performed at block 42 of Figure 4 comprises the step of comparing a peak location (i.e. the range at which a peak is located) in the range profile of the subspace component to the reference range value to determine whether its peak location is within a predefined threshold displacement range from the reference range value. If the peak location is within the predefined threshold displacement range from the reference range value, it is concluded that the component corresponds to a signal artefact.

The reference range value may be a known range between the first antenna element and the second antenna elements of the pair. However, in embodiments, the reference range value maybe predetermined based on multiple datasets captured by the medical examination system in a preliminary step. For example, the data acquisition method described above with respect to Figure 3 may be performed with the antenna array in plural, e.g. three, different rotational positions about the space which is to receive the breast. For a given transmit-and-receive antenna pair, the 1D output signals obtained at the rotational positions are compared in turn, such that the 1D output signal obtained at a first rotational position of the array is compared to a 1D output signal obtained at a second rotational position of the array, for example.

The 1D output signals from the plural measurements are transformed to the time domain, using an inverse fast Fourier transform (FFT), and the time domain data for each rotational position are cross-correlated against each other, e.g. the time domain data in the first rotational position is cross-correlated against the time domain data from the second rotational position, and optionally the time domain data in the third rotational position. It will be appreciated that the direct coupling signal will be consistent between the different rotational positions of the array, for a given transmit-and-receive antenna pair, and thus would result in the highest correlation when the two signals are cross correlated. Thus the range value at the point of maximum correlation between the datasets is used as the reference range value for the determination at block 42 of Figure 4.

Generating a compensated 1D output signal

As stated above, having identified components which correspond to the DC signal artefact, the method (at block 44 of Figure 4) generates a compensated 1D output signal to increase a relative intensity of components which correspond to the region of interest, where the relative intensity is with respect to the intensity of the direct coupling component(s).

This is achieved by associating weighting factors to respective subspace components, wherein the weighting factor for a given subspace component is determined based on whether the subspace component has been determined to correspond to the signal artefact or the region of interest. Those weighting factors are then applied to the plurality of subspace components to generate a plurality of weighted subspace components, which are then combined to generate a compensated one-dimensional output signal.

The numerical value of a weighting factor associated with a subspace component that corresponds to the signal artefact is less than the value of a weighting factor associated with a subspace component that corresponds to the region of interest.

In the present embodiment, the artefact components are dampened in that each component that corresponds to the artefact are associated with a weighting factor having a value of less than one, whereas components for the region of interest are associated with a weighting factor having a value of one. However, in alternative arrangements, components corresponding to the artefact may be associated with a weighting factor having a value of one (or less than one), whereas a component corresponding to the region of interest may be boosted by associating it with a weighting factor having a value of more than one.

In arrangements where an artefact component is dampened, that component may be associated with a weighting factor of zero, to effectively remove the component entirely. However, upon a true measured dataset, components that in reality correspond to the region of interest could happen to lie within the predefined threshold displacement range of the reference range profile and be inadvertently flagged as corresponding to the DC artefact. Therefore, the quality and accuracy of the compensated 1D output signal may be improved by associating the component with a weighting factor having a value between zero and one.

In this embodiment, however, the specific weighting factor for a component identified as corresponding to the DC artefact is determined based on an extent of similarity between the component (range profile) and the DC artefact model (reference range profile). The weighting factor has an inverse relationship with the extent of similarity such that components that are similar to the artefact model will be given less weight in the compensated 1D output signal than components that are less similar to the artefact model.

Where the determination at block 42 of Figure 4 generates a cross-correlated profile which represents the displacement of a peak in the component range profile relative to the peak of a reference range profile, the intensity of a peak in the cross-correlated profile is used to appropriately weight the component. The weighting, $W_l$, for a component identified as corresponding to the DC artefact is calculated as:

$$W_l = 1 - I_l \qquad (10)$$

Where $I_l$ is the maximum peak intensity of the cross-correlated profile generated for an individual component.

The weighting factor may take any value between "0" and "1", depending on the extent of similarity of the shape of the peaks, as indicated by $I_l$.

In the simulated example of Figure 10, the max intensity of the cross-correlated peak 100 is "0.99", such that component 1 will be assigned a weighting factor of "0.01". Component 2, however, is not dampened but is instead assigned a weighting factor of "1".

The compensated 1D output signal may be generated by multiplying the $\Phi_l$ values of a component by its associated weighting factor and summing the weighted $\Phi_l$ values for the plural components according to equation 8 (where $\Phi_l$ values of equation 8 are replaced by weighted values of $\Phi_l$). In this embodiment, however, the weighting factors are applied to the group of singular values generated at equation 4, as follows:

$$\mathbf{S}_w(l,l) = S(l,l)W(l) \tag{11}$$

thereby forming a group of weighted singular values in rectangular diagonal matrix form, $\mathbf{S}_w$.

The **U** matrix, the weighted singular value matrix, $\mathbf{S}_w$, and the complex conjugate transpose of **V,** are then multiplied to form the compensated 1D output signal, $\mathbf{X}_c$, in matrix form, as follows:

$$\mathbf{X}_c = \mathbf{U}\mathbf{S}_w\mathbf{V}^H \tag{12}$$

The compensated 1D output signal is then converted to its original data format through the concatenation of the matrix $\mathbf{X}_c$, as follows:

$$\Phi_c = \left( \left[ X_{c_{1,L}}, X_{c_{1,L-1}}, \cdots, X_{c_{1,1}} \right], \left[ X_{c_{2,1}}, X_{c_{3,1}}, \cdots, X_{c_{J,1}} \right] \right) \tag{13}$$

where $\Phi_c$ is the compensated 1D output signal.

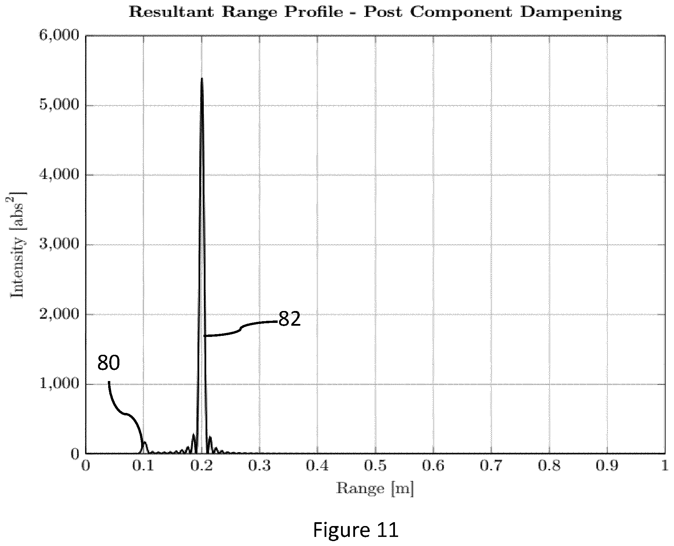

**Figure 11** is a plot showing the range profile of a compensated 1D output signal for the simulated data example referred to above with respect to Figures 8 to 10. As shown, the signal artefact 80 arising from component 1 (the direct coupling response) has been dampened such that the peak 82 arising from component 2 (the region of interest) is significantly greater than the peak 80 arising from component 1 (the direct coupling artefact). That is, the relative intensity of the DC component (component 1 corresponding to peak 80) as compared to the intensity of component 2 (peak 82) is significantly smaller in the compensated 1D output signal (Figure 11) than it is in the original 1D output signal (Figure 8).

It will be appreciated that the method has been described above with respect to associating a weighting factor to each subspace components of the plurality of subspace components. However, in alternative embodiments, only those components that have been identified as belonging to the DC artefact are associated with a weighting factor, and in those cases the weighting factor has a value of less than one. In other alternative embodiments, only components of interest (i.e. those that have been identified as belonging to the region of interest) are associated with a weighting factor, and in those cases the weighting factor has a value of more than one.

It can be seen from the above that the present invention provides a useful method of compensating for signal artefacts arising in 1D output signals generated by a microwave-based medical examination method.

For ease of explanation, the method has been described above with respect to the direct coupling artefact only. However, the method can be used for any type of signal artefact which is suspected to be present in the original 1D output signal and which can be predetermined and modelled, such as artefacts arising from skin reflections (which typically occur at a predictable range from each antenna element of a pair).

Further, although the system and data acquisition method has been described above with respect to a multistatic arrangement, this is not required. The invention is applicable to quasi-monostatic arrangements in which two antenna elements are positioned close together, e.g. adjacent or proximal, such that the data set is considered a monostatic one. The invention is also applicable to monostatic arrangements, in which a single antenna element transmits and receives the microwave energy during an acquisition period. That is, the system may comprise a single antenna element which is connected to the transmit path and the receive path of the system during an acquisition period. However, the system may instead comprise a plurality of antenna elements but only a first antenna element of the plural antenna elements transmits and receives the microwave signal during an acquisition period. In such monostatic arrangements, the 1D output signal represents the measured microwave energy (scattering parameters) detected at a single antenna element during an acquisition period, i.e. the same antenna element which transmitted the microwave energy. The data acquisition method may comprise a single acquisition period, rather than a plurality of such periods as is described with respect to Figure 3.

The invention is also applicable to bistatic arrangements in which a first antenna element is connected to the transmit path while a second, different antenna element of the system is connected to the receive path during an acquisition period. In those arrangements, the system may comprise only two antenna elements.

While the invention has been described above with respect to embodiments in which the reference data has been predetermined for the signal artefact, and therefore represents an aspect of the signal response predicted to arise from the signal artefact (such as direct coupling between the antenna pair), this is not required. The reference data, i.e. the reference range profile or reference range value, may instead be predetermined with respect to the region of interest, specifically a target region in which a tumour or other pathology is expected. For example, where the region of interest is a target region in which a possible tumour or other pathology is expected, the position or range of that target region can be predicted and a suitable reference range profile or reference range value can be predetermined based thereon.

The reference range profile for the region of interest may indicate an expected profile of signal intensity values at a range at which the target region is expected to be. In such cases, the range profile of the components may be compared to the reference range profile and the component may be determined to correspond to the region of interest if its range profile satisfies a match criterion indicating that it is sufficiently similar to the reference data for the region of interest. This may be done in a manner substantially as described above, e.g. by performing a cross-correlation between the component range profile and the reference range profile, and determining that the component corresponds to the region of interest if the cross-correlated profile is within a predefined threshold displacement range from the origin of the cross-correlated domain. Further, appropriate weighting values may be determined for components that have been determined to correspond to the region of interest, based on an intensity value of a peak in the cross-correlated profile. For example, the weighting factor for the subspace component that has been determined to correspond to the region of interest may be proportional to an intensity value of a peak in the cross-correlated profile, when the range profile and the reference range profile are normalised prior to the cross-correlation.

Where the reference data comprises a reference range value for the region of interest, i.e. a range value (from the first and/or second antenna elements) at which the region of interest is expected to be. In such cases, a peak of the component range profile is compared to the reference range value to determine whether its peak location is within a predefined threshold displacement range from the reference range value. An appropriate weighting may then be associated with the component, based on whether or not the peak location is within a predefined threshold displacement range.

Although the method of the present invention has been described above in the context of a microwave-based antenna system, it will be appreciated that the method may be used in radiofrequency antenna systems more generally. For example, other bandwidths of the electromagnetic spectrum, specifically those below the microwave range, could be used instead of the microwave bandwidth.

Further, while the method has been described as being used in the medical imaging field, the method could instead be applied to any antenna-based imaging system, such as those used in "through-the-wall" or "ground-penetrating" radar applications, where the object to be investigated is not necessarily an anatomical object. Further still, the method need not be applied in a system configured to generate image data, but could instead be applied to any radiofrequency antenna system which has a set of one or more antenna elements, and which is suitable for generating a 1D output signal representing the detected radiofrequency wave energy. The advantages of the invention, i.e. the improvement in accuracy of the 1D output signal, may be realised regardless of how the compensated 1D output signal is to be used.

Finally, it will be appreciated that the means for rotating the antenna array is not essential to the invention. The option to rotate the array may be provided with the intention of it being used in the event that additional data sets are required to achieve a desired level of image quality, methods for which are not within the scope of this disclosure. For example, further artefact reduction may be achieved using additional datasets taken with the array at different positions.

To avoid unnecessary duplication of effort and repetition of text in the specification, certain features are described in relation to only one or several aspects or embodiments of the invention. However, it is to be understood that, where it is technically possible, features described in relation to any aspect or embodiment of the invention may also be used with any other aspect or embodiment of the invention.

The invention is not limited to the embodiments described herein and may be modified or adapted without departing from the scope of the present invention.

**Claims**

1. A method, in a microwave-based medical examination system comprising a set of one or more antenna elements in operative proximity to a space which includes an object to be investigated, wherein the method comprises:

   energising a first antenna element of the set to transmit microwave energy towards the space so as to irradiate the object;

detecting, at the first antenna element or a second antenna element of the set, the transmitted microwave energy returned back from the space including the object;

generating a one-dimensional output signal representing the detected microwave energy;

separating the one-dimensional output signal into a plurality of subspace components;

determining, for each one of the plurality of subspace components, whether the subspace component corresponds to a signal artefact or a region of interest within the space, based on a comparison of the subspace component to predetermined reference data; and

processing the one-dimensional output signal to increase a relative intensity of one or more subspace components that have been determined to correspond to the region of interest, wherein the relative intensity is with respect to an intensity of a subspace component that has been determined to correspond to the signal artefact, thereby generating a compensated one-dimensional output signal.

2. The method of claim 1, wherein separating the one-dimensional output signal into a plurality of subspace components comprises:

generating a matrix representation of the one-dimensional output signal;

performing a singular value decomposition to factorise the matrix in terms of its singular vectors and a group of singular values;

generating, for each singular value of the group, a component matrix by reversing the singular value decomposition with respect to the singular value, wherein each component matrix represents a respective subspace component of the one-dimensional output signal; and

optionally concatenating the matrix representation.

3. The method of claim 2, wherein the matrix representation is a Hankel matrix representation.

4. The method of any preceding claim, wherein determining whether the subspace component corresponds to a signal artefact or a region of interest comprises:

generating a range profile of the subspace component; comparing the range profile to the reference data; and concluding that the subspace component corresponds to the signal artefact or the region of interest based on whether or not the range profile satisfies a match criterion indicating that it is sufficiently similar to the reference data.

5. The method of claim 4, wherein:
the reference data is in the form of a reference range profile for the signal artefact or the region of interest;

comparing the range profile to the reference data comprises performing a cross-correlation of the range profile and the reference range profile, to generate a cross-correlated profile; and

the range profile satisfies the match criterion if the cross-correlated profile has a peak location within a predefined threshold displacement range from an origin of the cross-correlated domain.

6. The method of claim 4, wherein:

the reference data is in the form of a reference range value for the signal artefact or the region of interest; and

the range profile satisfies the match criterion if the range profile has a peak location within a predefined threshold displacement range from the reference range value.

7. The method of claim 5 or 6, wherein the predefined threshold displacement range is calculated as: $\frac{v}{B}$ , where B is the bandwidth of the transmitted microwave energy and v is its speed of propagation.

8. The method of any preceding claim, wherein processing the one-dimensional output signal to increase a relative intensity of one or more subspace components that have been determined to correspond to the region of interest comprises:

associating the plurality of subspace components with respective weighting factors, wherein the weighting factor for a subspace component that has been determined to correspond to the signal artefact is less than the weighting factor for a subspace component that has been determined to correspond to the region of interest;

applying the weighting factors to the plurality of subspace components to generate a plurality of weighted subspace components; and
combining the plurality of weighted subspace components to generate the compensated one-dimensional output signal.

9. The method of claim 2 or 3, wherein processing the one-dimensional output signal to increase a relative intensity of one or more subspace components that have been determined to correspond to the region of interest comprises:

associating the plurality of subspace components with respective weighting factors, wherein the weighting factor for a subspace component that has been determined to correspond to the signal artefact is less than the weighting factor for a subspace component that has been determined to correspond to the region of interest; applying the weighting factors to the group of singular values to generate a weighted group of singular values; and reversing the singular value decomposition by combining the singular vectors and the weighted group of singular values.

10. The method of claim 8 or 9 when dependent on claim 5, wherein:

the reference data is in the form of a reference range profile for the signal artefact; and
the weighting factor for the subspace component that has been determined to correspond to the signal artefact is inversely related to an extent of similarity between the subspace component and the reference range profile for the signal artefact.

11. The method of claim 10, wherein:

the range profile and the reference range profile are normalised prior to the cross-correlation; and
the weighting factor for the subspace component that has been determined to correspond to the signal artefact is calculated as: $1 - I_l$, wherein $I_l$ is an intensity value of a peak in the cross-correlated profile.

12. The method of any preceding claim, wherein:

the transmitted microwave energy is detected at the second antenna element;
the predetermined reference data represents an aspect of the microwave energy that is expected to be detected at the second antenna element as a result of direct coupling between the first antenna element and the second antenna element.

13. The method of any preceding claim, wherein the reference data comprises a reference range profile in the form of a point spread function centred at a predetermined range at which the signal artefact or the region of interest is expected.

14. The method of any preceding claim, wherein the one-dimensional output signal represents the microwave energy detected by the first antenna element or the second antenna element only, during an acquisition period in which only the first antenna element transmits the microwave energy.

15. A microwave-based medical examination system comprising:

a set of one or more antenna elements in operative proximity to a space for receiving an object to be imaged; and
processing circuitry configured to perform the method of any preceding claim.

Figure 1

Figure 2

Figure 3

```
┌─────────────────────────────┐
│   Separate one-dimensional   │
│   output signal into individual │──── 40
│     subspace components      │
└─────────────────────────────┘
               │
               ▼
┌─────────────────────────────┐
│     Determine if subspace    │
│      components of signal    │
│  correspond to signal artefact or │──── 42
│       a region of interest    │
└─────────────────────────────┘
               │
               ▼
┌─────────────────────────────┐
│    Generating a compensated   │──── 44
│   one-dimensional output signal │
└─────────────────────────────┘
```

Figure 4

Phase Domain Profile

Figure 5

Range Profile

Figure 6

| Generate Hankel matrix of one-dimensional output signal | — 70 |

| Perform singular value decomposition of Hankel matrix | — 72 |

| Isolate subspace components to generate a component matrix for each component | — 74 |

| Concatenate the first row and end column of the isolated component matrix | — 76 |

Figure 7

Figure 8

Figure 9

Figure 10

Figure 11

| | Europäisches Patentamt European Patent Office Office européen des brevets | **EUROPEAN SEARCH REPORT** | Application Number EP 21 20 5172 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ELAHI MUHAMMAD ADNAN ET AL: "ARTIFACT REMOVAL ALGORITHMS FOR MICROWAVE IMAGING OF THE BREAST", PROGRESS IN ELECTROMAGNETICS RESEARCH, vol. 141, 1 January 2013 (2013-01-01), pages 185-200, XP055900985, DOI: 10.2528/PIER13052407 Retrieved from the Internet: URL:https://www.jpier.org/PIER/pier141/11.13052407.pdf> * abstract * * sections 2.3.3, 2.5, 3 * * the whole document * | 1-4,8-15 | INV. A61B5/0507 A61B5/00 |
| X | OSCAR GOMEZ ED – OSCAR GOMEZ: "MIMO Radar with Colocated Antennas: Theoretical Investigation, Simulations and Development of a Experimental Platform", PHD THESIS TO OBTAIN THE TITLE OF PH.D. OF SCIENCE OF THE UNIVERSIT E PARIS-EST SPECIALTY: ELECTRONICS, OPTRONICS AND SYSTEMS, UNIVERSIT E PARIS-EST DOCTORAL SCHOOL MSTIC, FR, PAGE(S) 1 - 172 , 16 June 2014 (2014-06-16), XP009528557, Retrieved from the Internet: URL:https://www.researchgate.net/publication/278190031_MIMO_Radar_with_Colocated_Antennas_Theoretical_Investigation_Simulations_and_Development_of_an_Experimental_Platform?channel=doi&linkId=557d2a6d08aeb61eae23665d&showFulltext=true * sections 1.1 and 4.2 * * the whole document * | 1,4-15 | |
| A | US 2019/175095 A1 (BORE CHRIS [GB]) 13 June 2019 (2019-06-13) * figure 2 * * the whole document * | 1-15 | TECHNICAL FIELDS SEARCHED (IPC) A61B |

The present search report has been drawn up for all claims

| Place of search The Hague | Date of completion of the search 15 March 2022 | Examiner Sarcia, Regis |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 20 5172

This annex lists the patent family members relating to the patent documents cited in the above-mentioned European search report.
The members are as contained in the European Patent Office EDP file on
The European Patent Office is in no way liable for these particulars which are merely given for the purpose of information.

15-03-2022

| Patent document cited in search report | | Publication date | Patent family member(s) | | Publication date |
|---|---|---|---|---|---|
| US 2019175095 | A1 | 13-06-2019 | EP | 3281583 A1 | 14-02-2018 |
| | | | GB | 2552837 A | 14-02-2018 |
| | | | JP | 2019531773 A | 07-11-2019 |
| | | | US | 2019175095 A1 | 13-06-2019 |
| | | | WO | 2018029488 A1 | 15-02-2018 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009060181 A

**Non-patent literature cited in the description**

- **R. C. CONCEIÇÃO ; J. J. MOHR ; M. O'HAL-LORAN.** *An Introduction to Microwave Imaging for Breast Cancer Detection,* July 2016, 49-50